# EUROPEAN PATENT APPLICATION

(11) **EP 3 926 322 A1**
(43) Date of publication of application: **22.12.2021**
(21) Application number: 20777735.0
(22) Date of filing: 26.03.2020
(51) Int. Cl.: G01N 1/02, G01N 21/78

(54) **INTEGRATED DEVICE FOR MEDICAL SAMPLING AND TESTING**

(30) Priority: 27.03.2019 CN 201910237489
(71) Applicant: Jiangsu Gemkeeper Biotech Co., Ltd., Jiangsu 214000 (CN)
(72) Inventor: LIU, Yuan, Xianyang, Shaanxi 713703 (CN)
(74) Representative: karo IP
(86) International application number: PCT/CN2020/081282
(87) International publication number: WO 2020/192711

(57) **Abstract**

An integrated device for medical sampling and testing, comprising a sampling rod (15) and a casing. A test paper accommodating cavity (2) and a diluent cavity (5) are comprised in the casing. A liquid outlet (4) is provided at a bottom part of the diluent cavity (5), and a hole-plugging object (14) is provided on the liquid outlet (4). The sampling rod (15) is connected to a plunger (16), and the plunger (16) is placed in the diluent cavity (5). Two ends of the sampling rod (15) are end A (8) and end B (18) respectively. By pushing end B (18) of the sampling rod (15), as the plunger (16) is pushed toward end A (8) of the sampling rod (15) in the diluent cavity (5), the sampling rod (15) forces out the hole-plugging object (14) at the liquid outlet (4). The preset device has a simple structure, and may simultaneously achieve the sampling, dilution and testing of feces. Moreover, the device may integrate the collection, dilution, mixing, sample addition and testing of a feces sample, so that an ordinary person may screen people at home who are at high-risk for bowel and stomach cancer, which is of great social significance. The device has a clever structure, is easy to use, and also ensures the pre-filled diluent is sealed in.

## Description

### FIELD OF TECHNOLOGY

The present invention belongs to the field of medical reagent detection.

### BACKGROUND

Routine fecal occult blood test should be carried out through the collection of the stool to inspection by the tested subject and then being tested by the professionals in hospital. However, the stool sample was not inspected frequently, because of the high requirements of stool collection, the preservation and transportation of specimen, and the neglect or no intention of the tested subject during the medical examination in the hospital. Thus, fecal examination is the item with the highest abandon rate in our country and the word. The fecal examination is the first recommended method for colorectal cancer screening according to World Health Organization (WHO), American Society of Clinical Oncology (ASCO) and the consensus of China expert. At present, the specificity of stool tests for colorectal cancer is close to 80%, yet the specificity of blood tests for colorectal cancer is only about 30%.

At present, there is no device suitable for self-test of fecal occult blood on the market. Some manufacturers who product the gold labeled immunoassay reagent for testing fecal occult blood provide excrement collector. But aforesaid excrement collector has neither sampling control device nor detector. Meanwhile, all tests must open fecal fluid. At present, fecal occult blood tests are all limited to be carried out in professional medical institutions. Specialized examination personnel generally first put the stool in a container with buffer solution to dissolve, and then pour the feces in container into a small tubular container, and then insert the absorbent test paper into the small tubular container with the feces for detection.

Current detection process of fecal examination in hospital is shown below. The subject for the physical examination get the stool sample cup from the clinical laboratory, and then collect the stool sample in the cup to transport to the clinical laboratory. The testing person mixes the sample into the diluent and discards the redundant sample. Then the mixed sample is absorbed by pipette and added on the reagent mats. The results are identified after 5-10 minutes. During the entire process, the sample were exposed to the detection of the testing person, and it is easy to overturn and spill sample liquid, which causes contamination due to hundreds of species of bacteria in fecal sample. Meanwhile, the discarded sample is easy to cause secondary pollution.

After routine stool test and specimen collection, the examination should be completed within 1-2 hours; otherwise, due to the influence of pH and digestive enzymes and the cellular ingredient in stool can be destroyed and decomposed. The fecal sample is no possible to collect at any moment as the blood sample. Meanwhile the execution times that subject for the physical examination transport the collected sample from home to the hospital and whether the laboratory can test immediately are uncertain. For the foregoing reasons, it is easy to result in giving up taking a fecal examination. When the subject transports the fecal sample from home to the hospital, the insanitation, time delays and the embarrassment are major contributors to abandoning fecal examination.

### SUMMARY OF THE INVENTION

In order to overcome the shortcomings of existing technology, the present invention provides an integrated device for medical sampling and testing which brings more convenience for fecal occult blood test.

In order to achieve the above purpose, an integrated device for medical sampling and testing in present invention comprises a sampling rod and a casing. The casing comprises a test paper accommodating cavity and a diluent cavity. A liquid outlet is arranged at the bottom of the diluent cavity, and a hole-plugging object is arranged on the liquid outlet. The upper opening of the diluent cavity is communicated with the outside.

Further, it also includes a plunger; the plunger is placed in the diluent cavity. The plunger is equipped with a pore. The end A of the sampling rod can be detachably passed through the pore. The end B of the sampling rod is pushed so that the sampling rod along with the plunger can be propelled to the end A of the sampling rod in the diluent cavity to poke the hole-plugging object at the liquid outlet.

Further, the test paper accommodating cavity contains a test paper, and the sample pad of the test paper is arranged downward. The liquid flowing out from the liquid outlet can be in contact with the sample pad.

Further, a separation layer is formed between the test paper accommodating cavity and the diluent cavity. A communication cavity is arranged on the lower side of the separation layer. Both the liquid outlet and the bottom of the test paper accommodating cavity are connected to the communication cavity.

Further, the bottom of the casing is set as a detachable bottom cover. After the bottom cover is detached, the communication cavity is connected to the outside.

Further, the outer wall of end B of the sampling rod is movably fit with the inner wall of the diluent cavity. A limited buckle A is equipped on the outer wall of end B of the sampling rod, and a limited buckle B is equipped on the inner wall of the diluent cavity. The limited buckle A and the limited buckle B can clamp and limit each other.

Further, the diameter of the end A of the sampling rod is smaller than the inner diameter of the liquid outlet.

Further, the end A of the sampling rod is defined as a sampling end, and the sampling end is equipped with a threaded structure and is arranged downward.

Further, a reagent slot and a desiccant are also placed in the reagent containing cavity, and the test paper is placed in the reagent slot.

Further, the pre-installed diluent is located between the hole-plugging object and the plunger in the diluent cavity.

Beneficial effects: The device disclosed by the invention is simple in structure, which is characterized by integrating stool sample collection, dilution, mixing, sample addition, and testing of a feces sample. Ordinary people may screen people at home who are at high-risk for bowel and stomach cancer. The device in present invention is ingenious in structure and convenient to use, and also guarantees the pre-filled diluent is sealed in. Thus the present invention solves the core problems of fecal sampling, transportation, short storage time, and pollution. The colorectal cancer includes colon cancer and rectal cancer, which is one of the five major malignant tumors and has a very high incidence. Almost 80% of colorectal cancers are diagnosed at an advanced stage, and the mortality rate of colorectal cancer is also very high. The colon cancer without lymph node metastasis during early detection can be treated through surgical resection; the five-year survival rate can reach more than 90%. The invention is characterized by the simplicity of operator. The device in this invention can be popularized globally, and can effectively reduce the global mortality of intestinal cancer, which screening for bowel cancer can be done at home, which is of great social significance.

### DESCRIPTION OF THE DRAWINGS

Fig.1 depicts a cut-away schematic diagram of the overall structure of the device in present invention;
Fig.2 depicts a structural schematic diagram of the sampling rod in present invention;
Fig.3 depicts a structural schematic diagram of the casing;
Fig.4 depicts a structural schematic diagram on the basis of Fig. 2 with removing the plunger.

### DETAIL DESCRIPTION

The present invention will be further explained in conjunction with the drawing, as follows.

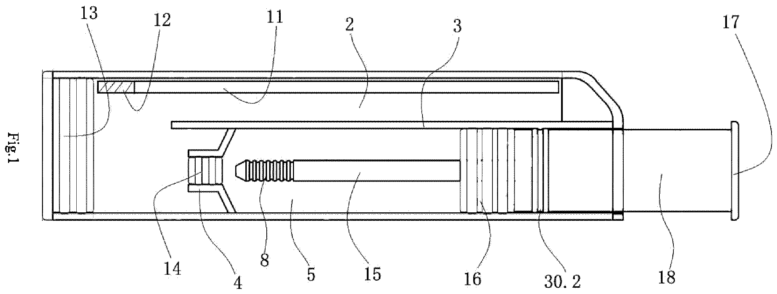

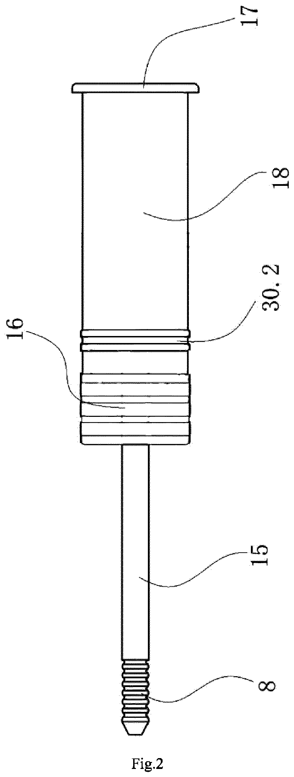

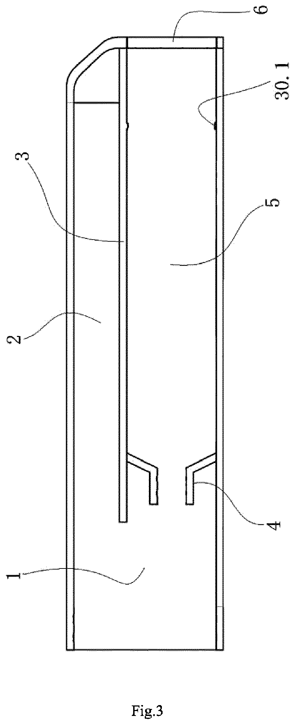

As shown in Fig. 1 to 4, an integrated device for medical sampling and testing includes a sampling rod 15 and a casing. The casing in this embodiment is a transparent plastic structure as shown in Fig.3; the casing comprises a test paper accommodating cavity 2 and a cylindrical diluent cavity 5; the bottom of the diluent cavity 5 is equipped with a liquid outlet 4, and the liquid outlet 4 is equipped with a hole-plugging object 14. The hole-plugging object 14 in this embodiment is cylindrical silicone material;

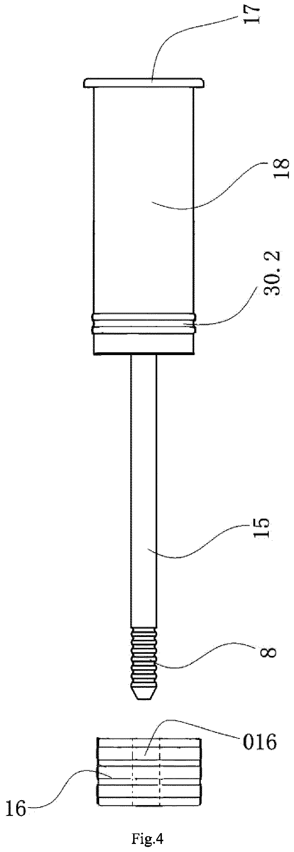

It also includes a plunger 16, which is placed in the diluent cavity 5, as shown in Fig.4. The plunger 16 in this embodiment also is cylindrical silicone material, and is equipped with a round pore 016 on its concentric location. In this embodiment, the end A of the sampling rod can pass downward through the round pore 016 of the plunger 16. The diameter of the round pore 016 is matched with the diameter of the end A 8 of the sampling rod. In addition, when the end A 8 of the sampling rod is threaded through the round pore 016, the excess feces on the thread can be scraped out, so as to achieve the effect of quantitatively collecting fecal samples.

The plunger 16 is coaxially and movably arranged in the diluent cavity 5 and closely fitted with the diluent cavity 5. In this embodiment, the diluent is pre-installed between the hole-plugging object 14 and the plunger 16 in the diluent cavity 5. The two ends of the sampling rod 15 of this embodiment are defined as the end A 8 and the end B 18 respectively. The end A 8 of this embodiment serves as a sampling end. The sampling end is equipped with a threaded structure and is arranged downward, which is used to stick stool samples. The outer diameter of end B 18 in this embodiment is compatible with the diluent cavity 5, and the top of the end B 18 in this embodiment is equipped with a pressing plate 17. The plunger 16 can be pushed downward by pressing the pressing plate 17. The sampling rod 15 can be pushed into the diluent cavity 5 with the plunger 16 to the end A 8 of the sampling rod 15, which implements that the hole-plugging object 14 at the liquid outlet 4 is poked, and the liquid flows out from the liquid outlet 4 to the communication cavity 1 with the effect of the pressure. In order to ensure that the end A 8 can smoothly poke the hole-plugging object 14 in the liquid outlet 4 and the liquid flow out smoothly from the liquid outlet 4, the diameter of the end A 8 of the sampling rod 15 in this embodiment is smaller than the inner diameter of the liquid outlet 4.

The test paper accommodating cavity 2 contains a test paper 11, and the sample pad 12 of the test paper 11 is arranged downward; the liquid flowing from the liquid outlet 4 can be in contact with the sample pad 12. In addition, to ensure that the degree of dryness and the fixing of the test paper of the test paper accommodating cavity 2, the reagent slot 05 and the desiccant 03 are also placed in the reagent containing cavity 2 in this embodiment, and the test paper 11 is stuck in the reagent slot 05.Between the test paper 11 and the desiccant 03 is the reagent slot 05, and the reagent slot 05 separates the test paper 11 from the desiccant 03.

A separation layer 3 is formed between the test paper accommodating cavity 2 and the diluent cavity 5, a communication cavity 1 is provided on the lower side of the separation layer 3, and both the liquid outlet 4 and the bottom of the test paper accommodating cavity 2 are connected to the communication cavity 1. The bottom of the casing in this embodiment is set as a detachable bottom cover 13; the bottom cover 13 seals the communicating cavity 1 at the bottom of the casing. After the bottom cover 13 is removed, the communicating cavity 1 communicates with the outside world. The bottom cover 13 with the detachable structure is convenient for pre-installing the reagent slot 05, the desiccant 03 and the test paper 11 before leaving the factory.

The outer wall of the end B 18 of the sampling rod 15 is movably matched with the inner wall of the diluent cavity 5, and the outer wall of the end B 18 of the sampling rod 15 is provided with a limited buckle A 30.2, which is made of two sections of plastic material with ring structure. The inner wall of the diluent cavity 5 is equipped with a limited buckle B 30.1. The limited buckle B 30.1 is made of two sections of plastic material matched with the limit buckle A 30.2. In the ex-factory state, the limited buckle A 30.1 and the limited buckle B 30.2 can clamp and limit each other because of that one of them is convex structure and the other one is concave structure. When using the device in this embodiment, the object presses hard or pulls the pressing plate 17 outward to make the plastic material of the limited buckle A 30.1 and the limited buckle B 30.2 clamping with each other deformable. Thereby, the limited buckle A 30.1 and the limit buckle B 30.2 are separated from each other, and the limit function is removed; the limited buckle A30. 1 and the limited buckle B 30.2 play a limit role of the sampling rod 15.

The specific operation method of this device:
In this embodiment, the diluent is pre-installed between the hole-plugging object 14 and the plunger 16 in the diluent cavity 5. First, keep the casing structure upright, hold the casing tightly with one hand, and then hold the end B 18 upwards to pull out the sampling rod 15. Subsequently, hold the end B 18 of the sampling rod 15 and use the threaded structure of the end A 8 of the sampling rod 15 to collect stool samples at multiple points, and then hold the end B 18 and pass the end A 8 of the sampling rod 15 through the round pore 016 on the plunger 16, the round pore 016 scrapes off the excess feces on the threaded structure of the end A 8 and the end A 8 of the sampling rod 15 blocks the round pore 016 so that the end A 8 of the sampling rod 15 is reinserted into the diluent cavity 5 and contacts with the diluent. At the same time, the limited buckle B30.1 and the limited buckle A30.2 are stuck with each other. At this time, start to shake the diluent cavity 5 so that the stool sample adhering on the end A of the sampling rod 15 is fully mixed with the diluent in the diluent cavity 5. Then continue to press the pressing plate 17 downward, and at the same time the limited buckle B30.1 is separated from the limited buckle A30.2. Then the sampling rod 15 is pushed into the diluent cavity 5 with the plunger 16 to the end A 8 of the sampling rod 15, which results to poke the pore plugging material 14 at the liquid outlet 4. Subsequently, the liquid flows out from the liquid outlet 4 to the communication cavity 1. At the same time, the downward movement of the plunger 16 increases the pressure in the diluent cavity 5. When the end A 8 is opened the pore plugging material 14 at the liquid outlet 4, the diluent mixed with the sample in the diluent cavity 5 will quickly gush out through the gap between the end A of the sampling rod and the liquid outlet 4 and flow into the communication cavity 1. At this time, the end A of the sample rod 15 is still placed in the liquid outlet 4, and the liquid which quickly flows through the liquid outlet 4 can also flush the residual feces on the end A of the sampling rod 15, so that the stool sample adhering on the end A of the sampling rod 15 is thoroughly washed out, thereby effectively ensuring that there is a sufficient amount of stool sample in the diluent. Subsequently, the liquid flowing into the communication cavity 1 will quickly infiltrate the sample pad 12 at the lower end of the test paper 11, and the liquid will slowly rise under the action of chromatography, and finally all contact with the test paper. The sample reacts with the test paper for 5-10 minutes and the chromatographic reaction result can be identified by the test paper. There is a C line and T mark on the test paper, and only one C line revealed is negative. If C line and T mark are all displayed, the result is shown as positive. If neither of C line and T mark is displayed, it means the test has failed. Thus the collection, dilution, sample addition, and detection can be integrated.

The above are only the preferred embodiments of the present invention. It should be pointed out that for those of ordinary skill in the art, without departing from the principle of the present invention, several improvements and modifications can be made, and all changes which come within the meaning and range of equivalency of the claims are therefore intended to be embraced therein.

## Claims

1. An integrated device for medical sampling and testing, which is **characterized in that** it comprises a sampling rod (15) and a casing; the housing includes a test paper accommodating cavity (2) and a diluent cavity (5); a liquid outlet (4) is arranged at the bottom of the diluent cavity (5), and a hole-plugging object (14) is arranged on the liquid outlet (4); the upper opening (6) of the diluent cavity (5) is communicated with the outside;
two ends of the sampling rod (15) are respectively an end A (8) and an end B (18); push the end B (18) of the sampling rod (15), and the sampling rod (15) can be propelled to the end A (8) of the sampling rod (15) in the diluent cavity (5) to poke the hole-plugging object (14) at the liquid outlet (4), and the liquid flows out from the liquid outlet (4) into the communication cavity (1) and is in connect with the sample pad (12).

2. An integrated device for medical sampling and testing according to Claim 1, which is **characterized in that** it further includes a plunger (16); the plunger (16) is placed in the diluent cavity (5); the plunger (16) is equipped with a pore (016); the end A of the sampling rod (15) can be detachably passed through the pore (016); the end B (18) of the sampling rod (15) is pushed so that the sampling rod (15) along with the plunger (16) is propelled to the end A (8) of the sampling rod (15) in the diluent cavity (5) to poke the hole-plugging object (14) at the liquid outlet (4).

3. An integrated device for medical sampling and testing according to Claim 1, which is **characterized in that** a separation layer (3) is formed between the test paper accommodating cavity (2) and the diluent cavity (5); a communication cavity (1) is arranged on the lower side of the separation layer (3); both the liquid outlet (4) and the bottom of the test paper accommodating cavity (2) are connected to the communication cavity (1).

4. An integrated device for medical sampling and testing according to Claim 1, which is **characterized in that** the bottom of the casing is set as a detachable bottom cover (13); after the bottom cover (13) is detached, the communicating cavity (1) is connected the outside.

5. An integrated device for medical sampling and testing according to Claim 1, which is **characterized in that** a test paper (11) a reagent slot (05) and a desiccant (03) are placed in the reagent containing cavity (2); the test paper (11) is placed in the reagent slot (05), and the sample pad (12) of the test paper (11) is arranged downward;

6. An integrated device for medical sampling and testing according to Claim 3, which is **characterized in that** the outer wall of the end B (18) of the sampling rod (15) is movably fit with the inner wall of the diluent cavity (5); a limited buckle A (30.2) is equipped on the outer wall of end B (18) of the sampling rod (15), and a limited buckle B (30.1) is equipped on the inner wall of the diluent cavity (5); the limited buckle A (30.1) and the limited buckle B (30.2) can clamp and limit each other.

7. An integrated device for medical sampling and testing according to Claim 1, which is **characterized in that** the diameter of the end A (8) of the sampling rod (15) is smaller than the inner diameter of the liquid outlet (4).

8. An integrated device for medical sampling and testing according to Claim 1, which is **characterized in that** the end A (8) of the sampling rod (15) is defined as a sampling end, and the sampling end is equipped with a threaded structure and is arranged downward.

9. An integrated device for medical sampling and testing according to Claim 1, which is **characterized in that** the pre-installed diluent is located between the hole-plugging object (14) and the plunger in the diluent cavity (5).
